# EUROPEAN PATENT APPLICATION

(11) **EP 4 223 780 A1**
(43) Date of publication of application: **09.08.2023**
(21) Application number: 21874554.5
(22) Date of filing: 29.09.2021
(51) Int. Cl.: C07K 16/28, A61K 39/395, A61P 35/00, C12N 15/13, C12N 15/63, C12N 5/10

(54) **ANTI-CD22 ANTIBODY MOLECULE OR ANTIGEN-BINDING FRAGMENT AND USE THEREOF**

(30) Priority: 29.09.2020 CN 202011048723
(71) Applicant: Kunming Sinoway Natural Pharmaceuticals Co., Ltd., Kunming, Yunnan 650217 (CN)
(72) Inventor: HAO, Zhenping, Kunming City, Yunnan 650217 (CN); ZHU, Jianwei, Shanghai 200240 (CN); XU, Li, Kunming City, Yunnan 650217 (CN); XIE, Yueqing, Frederick Maryland 21704 (US); JIANG, Hua, Frederick Maryland 21704 (US); HUANG, Haiqiu, Frederick Maryland 21704 (US)
(74) Representative: Schiweck Weinzierl Koch Patentanwälte Partnerschaft mbB
(86) International application number: PCT/CN2021/121826
(87) International publication number: WO 2022/068899

(57) **Abstract**

Provided in the present invention is an isolated anti-CD22 antibody or an antigen-binding fragment thereof, and further provided is the use thereof. The antibody or the antigen binding fragment thereof shows strong identification capability for and a high affinity to CD22 recombinant protein and a CD22 positive cell line, a strong internalization capability to enter the CD22 positive cell line, and an effective growth inhibition and cell apoptosis effect on the CD22 positive cell line after fusion with bacterial exotoxin.

## Description

### CROSS-REFERENCE TO RELATED APPPLICATIONS

The present patent application claims the priority benefit of Chinese patent application No. CN202011048723.0 filed on September 29, 2020, which is hereby incorporated by reference in its entirety.

### TECHNICAL FIELD

The present disclosure relates to the field of biomedicine, in particular to a novel humanized anti-CD22 antibody and application thereof.

### BACKGROUND OF THE INVENTION

CD22 (Siglec-2) is a sialic acid-binding immunoglobulin-like lectin (Siglec) receptor, that binds specifically to sialic acid (Sia)-containing glycans, facilitating cell adhesion and/or cell signaling. CD22 expression is restricted to B cells and plays a critical role in establishing a baseline level of B-cell inhibition, and thus is a critical determinant of homeostasis in humoral immunity. CD22 is expressed in neoplastic cells in various B-cell malignancies, including B-lymphocytic leukemias/lymphomas and mature B-cell leukemias/lymphomas. The expression of CD22 can be particularly strong in hairy cell leukemia (HCL) and prolymphocytic leukemia.

Studies have shown that anti-CD22 antibodies can be used in the treatment of autoimmune diseases and B cell tumors. In addition, anti-CD22 antibodies are often developed for conjugation to other drugs, such as cytotoxic agents, enabling the targeted delivery of the drugs to target cells by means of their targeting properties.

Accordingly, there is a need in the art for a novel anti-CD22 antibody or fragment thereof that has a high affinity for CD22 and can be used in the treatment of CD22-related diseases, either alone or when conjugated to other drugs or molecules.

### SUMMARY OF THE INVENTION

In order to solve the above technical problem, the present disclosure provides an isolated monoclonal antibody, which is a humanized anti-CD22 antibody that binds to CD22 and exhibits strong recognition ability and high affinity for recombinant CD22 protein and CD22-positive cells, strong internalization ability into CD22-positive cells, and potent growth inhibition and apoptosis induction effects on CD22-positive cells after fused with a bacterial exotoxin. Based on the isolated monoclonal antibody, the present disclosure provides various antibody formats comprising key domains of the antibody and corresponding uses thereof.

Technical solutions of the invention are as follows.

In one aspect, the present disclosure provides an isolated anti-CD22 antibody or antigen binding fragment thereof. As used herein, the "antigen binding fragment" of the antibody encompasses various functional fragments of the antibody that retain the binding ability of the antibody to CD22 and corresponding biological activities of the antibody.

With respect to sequence structure, the present disclosure provides an isolated anti-CD22 antibody or antigen-binding fragment thereof, comprising a heavy chain variable region (VH) and a light chain variable region (VL), wherein the heavy chain variable region (VH) and light chain variable region (VL) comprise respectively:
(1) H-CDR1 having an amino acid sequence as shown in SEQ ID NO. 19 (IYDMS), H-CDR2 having an amino acid sequence as shown in SEQ ID NO. 25 (YISSGGGTTYYPDSVKG), and H-CDR3 having an amino acid sequence as shown in SEQ ID NO. 21 (HSGYGTHWGVLFAY); and, L-CDR1 having an amino acid sequence as shown in SEQ ID NO. 22 (RASQDISNYLN), L-CDR2 having an amino acid sequence as shown in SEQ ID NO. 23 (YTSILHS), and L-CDR3 having an amino acid sequence as shown in SEQ ID NO. 24 (QQGNTLPWT); or
(2) H-CDR1 having an amino acid sequence as shown in SEQ ID NO. 19 (IYDMS), H-CDR2 having an amino acid sequence as shown in SEQ ID NO. 26 (YISSGGGTTYYPGSVKG), and H-CDR3 having an amino acid sequence as shown in SEQ ID NO. 21 (HSGYGTHWGVLFAY); and, L-CDR1 having an amino acid sequence as shown in SEQ ID NO. 22 (RASQDISNYLN), L-CDR2 having an amino acid sequence as shown in SEQ ID NO. 23 (YTSILHS), and L-CDR3 having an amino acid sequence as shown in SEQ ID NO. 24 (QQGNTLPWT).

The anti-CD22 antibody or antigen-binding fragment thereof provided by the present disclosure is capable of binding to antigen CD22, particularly human CD22, with high affinity.

Preferably, in the anti-CD22 antibody or antigen binding fragment thereof provided by the present disclosure, the heavy chain variable region may comprise an amino acid sequence as shown in SEQ ID NO: 4, SEQ ID NO: 3, SEQ ID NO: 6, or SEQ ID NO: 5, or an amino acid sequence having at least 75% identity to the amino acid sequence as shown in SEQ ID NO: 4, SEQ ID NO: 3, SEQ ID NO: 6, or SEQ ID NO: 5; and/or, the light chain variable region comprises an amino acid sequence as shown in SEQ ID NO: 2, SEQ ID NO: 7, or SEQ ID NO: 8, or an amino acid sequence having at least 75% of identity to the amino acid sequence as shown in SEQ ID NO: 2, SEQ ID NO: 7, or SEQ ID NO: 8.

The "at least 75% identity" as used in the context of the present disclosure with respect to sequence is any percent identity no less than 75%, such as at least 80%, preferably at least 85%, more preferably at least 90%, further preferably at least 91%, 92%, 93%, 94%, 95%, 96%, 97%, 98% or even 99% identity.

Further preferably, in the antibody or antigen binding fragment thereof provided by the present disclosure, the heavy chain variable region and the light chain variable region comprise respectively:
(1) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 2 or an amino acid sequence having at least 75% identity to the amino acid sequence as shown in SEQ ID NO: 2;
(2) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 7 or an amino acid sequence having at least 75% identity to the amino acid sequence as shown in SEQ ID NO: 7;
(3) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 7 or an amino acid sequence having at least 75% identity to the amino acid sequence as shown in SEQ ID NO: 7;
(4) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 8 or an amino acid sequence having at least 75% identity to the amino acid sequence as shown in SEQ ID NO: 8;
(5) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 8 or an amino acid sequence having at least 75% identity to the amino acid sequence as shown in SEQ ID NO: 8; or
(6) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 8 or an amino acid sequence having at least 75% identity to the amino acid sequence as shown in SEQ ID NO: 8.

In particular, the antibody or antigen binding fragment thereof provided by the present disclosure comprises at least a heavy chain variable region and a light chain variable region, both comprising the CDRs as described above and framework regions in an arrangement as: FR1-CDR1-FR2-CDR2-FR3-CDR3-FR4. Further alternatively, the up to 25% difference in amino acid sequence due to the "at least 75% identity" may be present in any framework region of the heavy chain variable region or the light chain variable region, or in any domain or sequence in the antibody or antigen binding fragment thereof provided by the present disclosure outside of the heavy chain variable region and the light chain variable region. The difference may result from amino acid deletion, addition or substitution at any position.

The antibody provided by the present disclosure is a murine antibody, a chimeric antibody or a fully or partially humanized antibody; alternatively, the antibody is in the form of a scFv, dsFv, (dsFv)₂, Fab, Fab', F(ab')₂ or Fv antibody. Preferably, the antibody is a monoclonal antibody or a single chain antibody.

In addition to the variable regions, the antibody or antigen-binding fragment thereof comprises a human or murine constant region, preferably a human or murine heavy chain constant region (CH) and/or light chain constant region (CL). Preferably, the antibody or antigen-binding fragment thereof comprises a heavy chain and a light chain; more preferably, the antibody or antigen binding fragment thereof comprises a heavy chain constant region selected from the group consisting of IgG, IgA, IgM, IgD and IgE and/or a kappa or lambda type light chain constant region. According to one particular embodiment of the invention, the antibody is a monoclonal antibody, preferably a murine, chimeric or humanized monoclonal antibody. According to one particular embodiment of the invention, the monoclonal antibody is an IgG1 antibody.

Further preferably, the antibody or antigen binding fragment thereof provided by the present disclosure comprises a heavy chain constant region comprising an amino acid sequence as shown in SEQ ID NO: 9 or an amino acid sequence having at least 75% identity to the amino acid sequence as shown in SEQ ID NO: 9; and/or a light chain constant region comprising an amino acid sequence as shown in SEQ ID NO: 10 or an amino acid sequence having at least 75% identity to the amino acid sequence as shown in SEQ ID NO: 10.

According to one particular embodiment of the invention, the present disclosure provides a monoclonal antibody having a heavy chain and a light chain comprising respectively:
(1) an amino acid sequence as shown in SEQ ID NO: 13 or an amino acid sequence having at least 75% identity to the amino acid sequence as shown in SEQ ID NO: 13; and, an amino acid sequence as shown in SEQ ID NO: 12 or an amino acid sequence having at least 75% identity to the amino acid sequence as shown in SEQ ID NO: 12;
(2) an amino acid sequence as shown in SEQ ID NO: 13 or an amino acid sequence having at least 75% identity to the amino acid sequence as shown in SEQ ID NO: 13; and, an amino acid sequence as shown in SEQ ID NO: 14 or an amino acid sequence having at least 75% identity to the amino acid sequence as shown in SEQ ID NO: 14;
(3) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 14 or an amino acid sequence having at least 75% identity to the amino acid sequence as shown in SEQ ID NO: 14;
(4) an amino acid sequence as shown in SEQ ID NO: 15 or an amino acid sequence having at least 75% identity to the amino acid sequence as shown in SEQ ID NO: 15; and, an amino acid sequence as shown in SEQ ID NO: 16 or an amino acid sequence having at least 75% identity to the amino acid sequence as shown in SEQ ID NO: 16;
(5) an amino acid sequence as shown in SEQ ID NO: 17 or an amino acid sequence having at least 75% identity to the amino acid sequence as shown in SEQ ID NO: 17; and, an amino acid sequence as shown in SEQ ID NO: 16 or an amino acid sequence having at least 75% identity to the amino acid sequence as shown in SEQ ID NO: 16; or
(6) an amino acid sequence as shown in SEQ ID NO: 18 or an amino acid sequence having at least 75% identity to the amino acid sequence as shown in SEQ ID NO: 18; and, an amino acid sequence as shown in SEQ ID NO: 16 or an amino acid sequence having at least 75% identity to the amino acid sequence as shown in SEQ ID NO: 16.

In another aspect, the present disclosure also provides a nucleic acid molecule comprising a nucleotide sequence encoding the anti-CD22 antibody or antigen-binding fragment thereof provided by the present disclosure, or encoding a heavy chain CDR, a light chain CDR, a heavy chain variable region, a light chain variable region, a heavy chain or a light chain comprised in the anti-CD22 antibody or antigen-binding fragment thereof.

In a further aspect, the present disclosure provides a vector comprising a nucleic acid molecule as described above. The vector can be a eukaryotic expression vector, a prokaryotic expression vector, an artificial chromosome, a phage vector and the like.

In yet a further aspect, the present disclosure provides a host cell comprising a nucleic acid molecule or vector as described above, or a host cell transformed or transfected with a nucleic acid molecule or vector as described above. The host cell may be any prokaryotic or eukaryotic cell, such as a bacterial, insect, fungal, plant or animal cell.

The antibody or antigen binding fragment thereof provided by the present disclosure can be obtained using any method known in the art. For example, the heavy chain variable region and/or the light chain variable region of the antibody, or the heavy chain and/or the light chain of the antibody, may be obtained from the nucleic acid molecule provided by the present disclosure, and then they may be assembled with the optional other domains of the antibody into an antibody; alternatively, the host cell provided by the present disclosure is cultured under conditions that allow the host cell to express the heavy chain variable region and/or the light chain variable region of the antibody or the heavy chain and/or the light chain of the antibody, and to assemble them into an antibody. Optionally, the method further comprises a step of recovering the produced antibody.

In another aspect, the present disclosure also provides a composition comprising an antibody or antigen binding fragment thereof, a nucleic acid molecule, a vector and/or a host cell provided by the present disclosure. Preferably, the composition is a pharmaceutical composition, which further optionally comprises a pharmaceutically acceptable carrier, adjuvant, and excipient.

In yet another aspect, the present disclosure also provides an immunoconjugate comprising the anti-CD22 antibody or antigen binding fragment thereof provided herein and a partner molecule which is a therapeutic agent. The therapeutic agent can be a cytotoxin (e.g., pseudomonas aeruginosa exotoxin (e.g., PE38)), a radioisotope, an antibiotic, a small molecule toxin, other drug, etc.

In addition, the present disclosure also provides a kit comprising an antibody or antigen binding fragment thereof, a nucleic acid molecule, a vector, the host cell, a composition, and/or an immunoconjugate provided by the present disclosure.

In a further aspect, the invention also provides use of an antibody or antigen binding fragment thereof, a nucleic acid molecule, a vector, the host cell, a composition, and/or a immunoconjugate provided herein for the manufacture of a medicament for the treatment of a disease which includes a tumor or cancer expressing CD22. For example, the disease is a CD22-related B cell malignant tumor, or a B cell malignant tumor characterized by high expression of CD22, such as CD22-high expressing lymphoma or leukemia. Preferably, the lymphoma is non-hodgkin's lymphoma, small lymphocytic lymphoma or mantle cell lymphoma; and the leukemia is chronic lymphocytic leukemia, hairy cell leukemia or acute lymphocytic leukemia.

Accordingly, the present disclosure provides a method for treating a disease, comprising administering to a subject in need thereof an antibody or antigen binding fragment thereof, a nucleic acid molecule, a vector, a host cell, a composition, and/or an immunoconjugate provided herein. The subject is a mammal, including a human or non-human primate, and a domestic, farm or laboratory mammal such as dog, cat, cow, pig, sheep, horse, mouse, rabbit, etc. Preferably, the subject is a human. The disease includes a tumor or cancer expressing CD22. For example, the disease is a CD22-related B cell malignant tumor, or a B cell malignant tumor characterized by high expression of CD22, such as CD22 high expressing lymphoma or leukemia. Preferably, the lymphoma is non-hodgkin's lymphoma, small lymphocytic lymphoma or mantle cell lymphoma; and the leukemia is chronic lymphocytic leukemia, hairy cell leukemia or acute lymphocytic leukemia.

### BRIEF DESCRIPTION OF THE DRAWINGS

Embodiments of the present invention are described in detail below with reference to the attached figures, in which:
Figure 1 shows detection results of binding of the antibodies provided by the present disclosure to CD22 by ELISA.
Figure 2 shows detection results of binding kinetics of the antibodies provided by the present disclosure to CD22 by SPR using 1:1 binding model, in which panel 2A: HA22 mAb; panel 2B: mAb-1; panel 2C: mAb-2; panel 2D: mAb-3; panel 2E: mAb-4; panel 2F: mAb-5; and panel 2G: mAb-6.
Figure 3 shows detection results of binding of the antibodies provided by the present disclosure to CD22 on cell membrane by FACS, in which panel 3A: Raji cells; panel 3B: REH cells; and panel 3C: CA46 cells.
Figure 4 shows detection results of endocytosis of the antibodies provided by the present disclosure by FACS, in which panel 4A: Raji cells; panel 4B: CA46 cells; and panel 4C: REH cells.
Figure 5 shows detection results of ADCC activity of the antibodies provided by the present disclosure, in which panel 5A: CA46 cells; panel 5B: Raji cells; and panel 5C: REH cells.
Figure 6 shows detection results of ADCC activity of the antibodies provided by the present disclosure at a high dose of 50 µg/mL, in which panel 6A: CA46 cells; panel 6B: Raji cells; and panel 6C: REH cells.

### DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

### Example 1 Construction and preparation of the antibodies

In this Example, an IgG1-type monoclonal antibody was reconstructed by obtaining VH and VL region sequences and fusing them to constant regions.

### 1.1 Humanization design

A framework shuffling strategy was used to alter amino acids in the framework regions of heavy chain variable region (V_{H}) and light chain variable region (V_{L}) comprised in the recombinant immunotoxin HA22 from the original murine to human ones. All the CDRs, which were determined using both the Kabat and Chothia numbering systems and the online tool (http://www.bioinf.org.uk/abs/), were left unchanged. Each of the framework and J regions in both the VH and VL of HA22 was aligned to the human antibody germline sequences in the international ImMunoGeneTics information system^{®} (IMGT: http://www.imgt.org) for the closest human antibody matches. Murine residues that were deemed as Vernier residues or important for VIFVL interactions were not changed to preserve the specificity and affinity towards CD22.

A panel of humanization designs were prepared and evaluated to select the final candidate that possessed maximum humanization while maintaining optimal affinity and efficacy profile.

### 1.2 Gene sequence determination

Amino acid sequences of the heavy chain variable region (VH) and light chain variable region (VL) contained in HA22 and humanized versions of the antibody are shown in Table 1. Elbow regions of the heavy and light chains were determined through defining antibody variable regions according to Kabat nomenclature, for subsequent mAb reconstruction; and CDR1, CDR2, and CDR3 were identified and underlined.

**Table 1. V region sequences in the heavy and light chains (CDRs are underlined)**

| Name | Sequence |
|---|---|
| HA22 VH | EVQLVESGGGLVKPGGSLKLSCAASGFAFSIYDMSWVRQTPEKCLEWV AYISSGGGTTYYPDTVKGRFTISRDNAKNTLYLQMSSLKSEDTAMYYC ARHSGYGTHWGVLFAYWGQGTLVTVSA (SEQ ID NO: 1) |
| HA22 VL | DIQMTQTTSSLSASLGDRVTISCRASQDISNYLNWYQQKPDGTVKLLIYY TSILHSGVPSRFSGSGSGTDYSLTISNLEQEDFATYFCQQGNTLPWTFGC GTKLEIK (SEQ ID NO: 2) |
| VH1 | EVQLVESGGGLVKPGGSLRLSCAASGFAFSIYDMSWVRQAPGKCLEWV AYISSGGGTTYYPDSVKGRFTISRENAKNSLYLQMNSLKSEDTAMYYC ARHSGYGTHWGVLFAYWGQGTMVTVSS (SEQ ID NO: 3) |
| VH2 | EVQLVESGGGLVKPGGSLRLSCAASGFAFSIYDMSWVRQTPEKCLEWVA YISSGGGTTYYPDSVKGRFTISRENAKNSLYLQMNSLKSEDTAMYYCAR HSGYGTHWGVLFAYWGQGTMVTVSS (SEQ ID NO: 4) |
| VH4 | EVQLVESGGGLVKPGGSLRLSCAASGFAFSIYDMSWIRQAPGKCLEWVA YISSGGGTTYYPGSVKGRFTISRENAKNSLYLQMNSLRAGDTAVYYCA RHSGYGTHWGVLFAYWGQGTMVTVSS (SEQ ID NO: 5) |
| VH5 | EVQLVESGGGLVKPGGSLRLSCAASGFAFSIYDMSWIRQAPGKCLEWVA YISSGGGTTYYPGSVKGRFTISRENAKNSLYLQMNSLRSEDTAVYYCAR HSGYGTHWGVLFAYWGQGTMVTVSS (SEQ ID NO: 6) |
| VL-2 | DIQMTQSTSTLSASVGDRVTITCRASQDISNYLNWYQQRPDGSVKLLIYY TSILHSGVPSRFSGSGSGTDYTLTISSLQDEDFATYFCQQGNTLPWTFGC GTKVEIK (SEQ ID NO: 7) |
| VL-4 | DIQMTQSPSTLSASVGDRVTITCRASQDISNYLNWFQQRPGQSPRLLIYY TSILHSGVPSRFSGSGSGTDYTLTISSLQPEDFATYYCQQGNTLPWTFGC GTKVEIK (SEQ ID NO: 8) |

### 1.3 Amino acid sequences of constant regions of IgG1

The antibody was determined to have a kappa-type light chain upon an analysis of the variable regions, so kappa-type light chain constant region and IgG1 heavy chain constant region were selected for the reconstruction of IgG antibodies. The constant region sequences are shown in Table 2.

**Table 2. Constant region sequences**

| Name | Sequence |
|---|---|
| CL |  (SEQ ID NO: 9) |
| CH1+Fc |  (SEQ ID NO: 10) |

### 1.4 Reconstructed IgG monoclonal antibodies

Complete heavy and light chains shown in Table 3 were reconstructed through fusing the V region sequences provided in Table 1 to the constant region sequences provided in Table 2.

**Table 3. Amino acid sequences of the reconstructed IgG antibodies (CDRs are underlined and constant regions are in italics)**

| Name | Combination | Sequence |
|---|---|---|
| HA22 mAb | HA22 HC | EVQLVESGGGLVKPGGSLKLSCAASGFAFSIYDMSWVRQ TPEKCLEWVAYISSGGGTTYYPDTVKGRFTISRDNAKNT LYLQMSSLKSEDTAMYYCARHSGYGTHWGVLFAYWGQ GTLVTVSAASTKGPSVFPLAPSSKSTSGGTAALGCLVKDYFP |
| | | EPVTVSWNSGALTSGVHTFPAVLQSSGLYSLSSVVTVPSSSLG TQTYICNVNHKPSNTKVDKKVEPKSCDKTHTCPPCPAPELL GGPSVFLFPPKPKDTLMISRTPEVTCVVVDVSHEDPEVKFN WYVDGVEVHNAKTKPREEQYNSTYRVVSVLTVLHQDWLNG KEYKCKVSNKALPAPIEKTISKAKGQPREPQVYTLPPSRDEL TKNQVSLTCLVKGFYPSDIAVEWESNGQPENNYKTTPPVLDS DGSFFLYSKLTVDKSRWQQGNVFSCSVMHEALHNHYTQKS LSLSPGK (SEQ ID NO: 11) |
| | HA22 LC | DIQMTQTTSSLSASLGDRVTISCRASQDISNYLNWYQQK PDGTVKLLIYYTSILHSGVPSRFSGSGSGTDYSLTISNLEQ EDFATYFCQQGNTLPWTFGCGTKLEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 12) |
| mAb-1 | V2 HC | EVQLVESGGGLVKPGGSLRLSCAASGFAFSIYDMSWVRQ TPEKCLEWVAYISSGGGTTYYPDSVKGRFTISRENAKNSL YLQMNSLKSEDTAMYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ KSLSLSPGK (SEQ ID NO: 13) |
| | HA22 LC | DIQMTQTTSSLSASLGDRVTISCRASQDISNYLNWYQQK PDGTVKLLIYYTSILHSGVPSRFSGSGSGTDYSLTISNLEQ EDFATYFCQQGNTLPWTFGCGTKLEIKRTVAAPSVFIFPPS |
| | | DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 12) |
| mAb-2 | V2 HC | EVQLVESGGGLVKPGGSLRLSCAASGFAFSIYDMSWVRQ TPEKCLEWVAYISSGGGTTYYPDSVKGRFTISRENAKNSL YLQMNSLKSEDTAMYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ KSLSLSPGK (SEQ ID NO: 13) |
| | V2 LC | DIQMTQSTSTLSASVGDRVTITCRASQDISNYLNWYQQR PDGSVKLLIYYTSILHSGVPSRFSGSGSGTDYTLTISSLQD EDFATYFCQQGNTLPWTFGCGTKVEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 14) |
| mAb-3 | V1 HC | EVQLVESGGGLVKPGGSLRLSCAASGFAFSIYDMSWVRQ APGKCLEWVAYISSGGGTTYYPDSVKGRFTISRENAKNS LYLQMNSLKSEDTAMYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN |
| | | GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ KSLSLSPGK (SEQ ID NO: 15) |
| | V2 LC | DIQMTQSTSTLSASVGDRVTITCRASQDISNYLNWYQQR PDGSVKLLIYYTSILHSGVPSRFSGSGSGTDYTLTISSLQD EDFATYFCQQGNTLPWTFGCGTKVEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 14) |
| mAb-4 | V1 HC | EVQLVESGGGLVKPGGSLRLSCAASGFAFSIYDMSWVRQ APGKCLEWVAYISSGGGTTYYPDSVKGRFTISRENAKNS LYLQMNSLKSEDTAMYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ KSLSLSPGK (SEQ ID NO: 15) |
| | V4 LC | DIQMTQSPSTLSASVGDRVTITCRASQDISNYLNWFQQRP GQSPRLLIYYTSILHSGVPSRFSGSGSGTDYTLTISSLQPE DFATYYCQQGNTLPWTFGCGTKVEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 16) |
| mAb-5 | V5 HC | EVQLVESGGGLVKPGGSLRLSCAASGFAFSIYDMSWIRQ APGKCLEWVAYISSGGGTTYYPGSVKGRFTISRENAKNS LYLQMNSLRSEDTAVYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ KSLSLSPGK (SEQ ID NO: 17) |
| | V4 LC | DIQMTQSPSTLSASVGDRVTITCRASQDISNYLNWFQQRP GQSPRLLIYYTSILHSGVPSRFSGSGSGTDYTLTISSLQPE DFATYYCQQGNTLPWTFGCGTKVEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 16) |
| mAb-6 | V4 HC | EVQLVESGGGLVKPGGSLRLSCAASGFAFSIYDMSWIRQ APGKCLEWVAYISSGGGTTYYPGSVKGRFTISRENAKNS LYLQMNSLRAGDTAVYYCARHSGYGTHWGVLFAYWGQ GTMVTVSSASTKGPSVFPLAPSSKSTSGGTAALGCLVKDYF PEPVTVSWNSGALTSGVHTFPAVLQSSGLYSLSSVVTVPSSSL GTQTYICNVNHKPSNTKVDKKVEPKSCDKTHTCPPCPAPEL LGGPSVFLFPPKPKDTLMISRTPEVTCVVVDVSHEDPEVKF NWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVL DSDGSFFLYSKLTVDKSRWQQGNVFSCSVMHEALHNHYTQ |
| | | KSLSLSPGK (SEQ ID NO: 18) |
| | V4 LC | DIQMTQSPSTLSASVGDRVTITCRASQDISNYLNWFQQRP GQSPRLLIYYTSILHSGVPSRFSGSGSGTDYTLTISSLQPE DFATYYCQQGNTLPWTFGCGTKVEIKRTVAAPSVFIFPPS DEQLKSGTASVVCLLNNFYPREAKVQWKVDNALQSGNSQES VTEQDSKDSTYSLSSTLTLSKADYEKHKVYACEVTHQGLSSP VTKSFNRGEC (SEQ ID NO: 16) |

### 1.5 Preparation of the monoclonal antibodies

pCDNA 3.4 vector was used as the expression vector for the mAbs. After codon optimization, full genes were synthesized and inserted into the pCDNA 3.4 vector and expression vectors for transfection were prepared using endotoxin-free plasmid preparation kit. ExpicCHO cell strain was used as a host for transient expression host. After transient transfection, cell culture supernatants were harvested, and antibodies were obtained through affinity purification, and then identified and stored at -80 °C.

### Example 2 Detection of binding of the antibodies to CD22 antigen

In this Example, ELISA plates were coated with CD22 antigen to capture antibodies to be evaluated, and the color developed through an HRP-labeled secondary antibody capable of specifically recognizing Fd region, and the binding (EC₅₀) of each mAb to the CD22 antigen was detected.

ELISA plates were coated with 200 ng/mL CD22 antigen (Biotinylated Human Siglec-2/CD22 Protein, Fc, Avitag^{™}, ACROBiosystems (ARCO)) at 100 µl per well, at 4 °C overnight. Next day, the plates were washed with PBST, then blocked by the added blocking buffer (PBST + 3% milk) at 37 °C for 1-2 hours, and then washed again with PBST.

Each antibody to be tested was diluted with PBST+1% BSA in gradient, to obtain antibody dilutions at concentrations of 0.01 ng/mL, 0.05 ng/mL, 0.25 ng/mL, 1.28 ng/mL, 6.40 ng/mL, 32 ng/mL, 160 ng/mL, 800 ng/mL, 4000 ng/mL, 20000 ng/mL, respectively; and 100 µl of each of the dilutions was added to each well of the ELISA plates which were then incubated at 37 °C for 1 hour.

After incubation, the plates were washed with PBST. 100 µl of 1:10000 diluted secondary antibody (Mouse anti-human IgG Fd secondary antibody, HRP; REF: SA5-10190, Invitrogen, USA) was added to each well of the ELISA plates which were then incubated at 37 °C for 1 hour. Thereafter, the plates were washed with PBST.

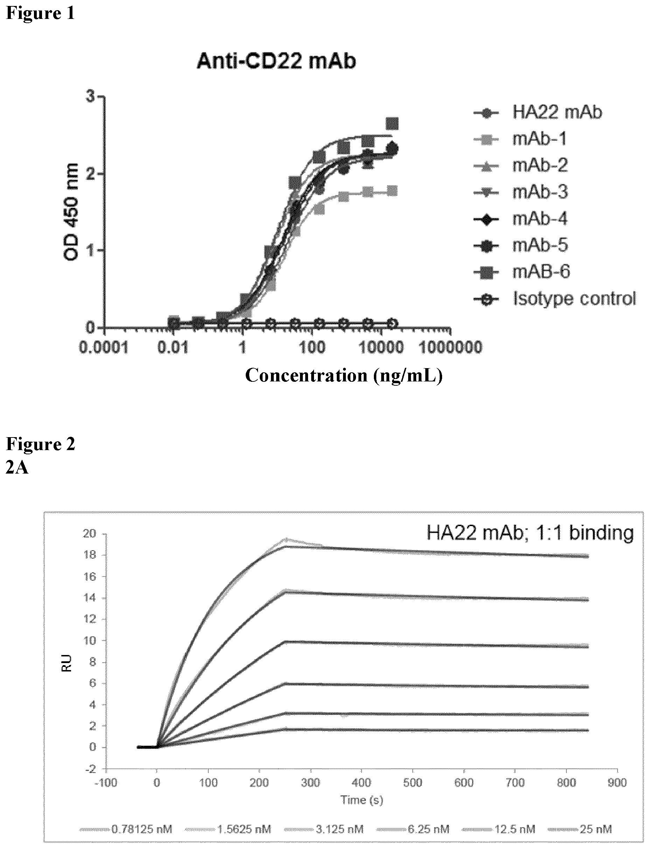

100 µl of TMB solution (TMB Single-Component Substrate solution, Cat#PR1200, Beijing Solarbio Science & Technology Co., Ltd.) was added to each well of the plates which were then incubated at room temperature for 15 minutes. Then 50 µl of stop buffer (ELISA stop buffer (10X), Cat#C1058, Beijing Solarbio Science & Technology Co., Ltd.) was added thereto, and the plates were shaken. Absorbance values at 450 nm were detected. Results are shown in Figure 1 and Table 4.

As shown in Figure 1 and Table 4, the OD450 value of the isotype control was below 0.1, indicating no interference on the results due to non-specific binding; the fitting degree of 4-parameter non-linear fitting was greater than 0.99, an excellent fitting degree, indicating the results can be analyzed objectively. The Top values showed mAb-6 exhibited a remarkable high signal value, reaching 2.435-2.564, which was significantly higher than the value 2.147-2.283 of HA22 mAb, indicating that mAb-6 has a higher plateau of saturation binding; the Top value of mAb-1 was 1.726-1.795, significantly lower than 2.147-2.283 of HA22 mAb; and the Top values of the remaining mAb-2, mAb-3, mAb-4, and mAb-5 were comparable to or slightly higher than that of HA22 mAb.

EC₅₀ value of the positive antibody HA22 mAb was 20.28 ng/mL, and EC₅₀ values of mAb-1 to mAb-6 were all lower than that of HA22 mAb, and the EC₅₀ values can be ranked as follows: mAb-2 < mAb-6 < mAb-3 < mAb-4 < mAb-1 < mAb-5 < HA22 mAb.

**Table 4. Detection results of binding of the antibodies to CD22 antigen by ELISA**

| | **EC₅₀ (ng/mL)** | | **R2** | | **Top** |
|---|---|---|---|---|---|
| HA22 mAb | 20.28 | HA22 mAb | 0.9935 | HA22 mAb | 2.147-2.283 |
| mAb-1 | 15.40 | mAb-1 | 0.9964 | mAb-1 | 1.726-1.795 |
| mAb-2 | 9.057 | mAb-2 | 0.9961 | mAb-2 | 2.202-2.293 |
| mAb-3 | 13.91 | mAb-3 | 0.9954 | mAb-3 | 2.176-2.278 |
| mAb-4 | 13.92 | mAb-4 | 0.9927 | mAb-4 | 2.191-2.329 |
| mAb-5 | 16.35 | mAb-5 | 0.9962 | mAb-5 | 2.221-2.325 |
| mAb-6 | 10.44 | mAb-6 | 0.9942 | mAb-6 | 2.435-2.564 |
| Isotype control | 0.1545 | Isotype control | 0.1733 | Isotype control | 0.05848-0.06417 |

### Example 3 Study on binding kinetics of the antibodies to CD22 antigen

In this Example, the binding kinetics of the antibodies to the antigen was detected by SPR.

Following reagents and materials were mainly used:
Antigen to be immobilized: CD22-Fc-biotinylated antigen: ACROBiosystems (ARCO)) SI2-H82F8;
Antibodies to be tested: Anti-CD22 mAbs;
Chip SA;
Immobilization buffer:
   (1) 1 M NaCl/50 mM NaOH;
   (2) 1 M NaCl/50 mM NaOH/50% isopropanol;
Running buffer: 1 x HBS-EP buffer;
Regeneration buffer: Glycine, pH 1.5.

Experimental steps were as follows:
1) A sensor chip SA was taken and its surface was cleaned with ultrapure water; and after dried under nitrogen, was inserted into the slot. The immobilization buffers (1) and (2) were added to the columns A1 and A2 in a 96 well plate, and 3.85 nM CD22-Fc-biotinylated antigen were added at 200 µL per well into corresponding wells in column A3, respectively. The program was set to immobilize the antigen, and the immobilization was stopped when delta RU of about 70 RU was achieved.
2) Program "binding test" was set, including association time of 250 s and dissociation time of 600 s, and a test was performed using HA22 mAb, a dissociation degree of 4-5% was shown.
3) The HA22 mAb was diluted to 1000 nM and subsequently to 100 nM, and further 2-fold diluted starting from 100 nM for 8 times, at a final volume of about 250 µL per well. The program was set up to detect the binding capacity of that antibody No. 1 to the antigen, with association time of 250 s and dissociation time of 600 s. The analysis software showed the binding of the HA22 mAb was fitted normally.
4) All the antibodies, i.e., mAb-1 to mAb-6, were diluted to 1000 nM and subsequently to 100 nM, and further 2-fold diluted starting from 100 nM for 8 times, at a final volume of about 250 µL per well. The same program as that in step 3 was set up to detect affinity in batches. Results were analyzed using Biacore 8K evaluation.

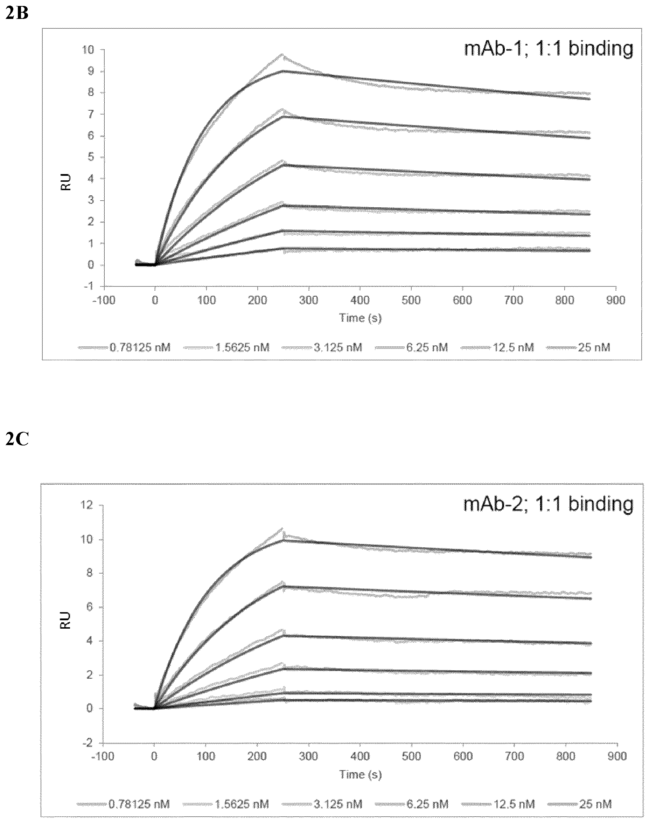

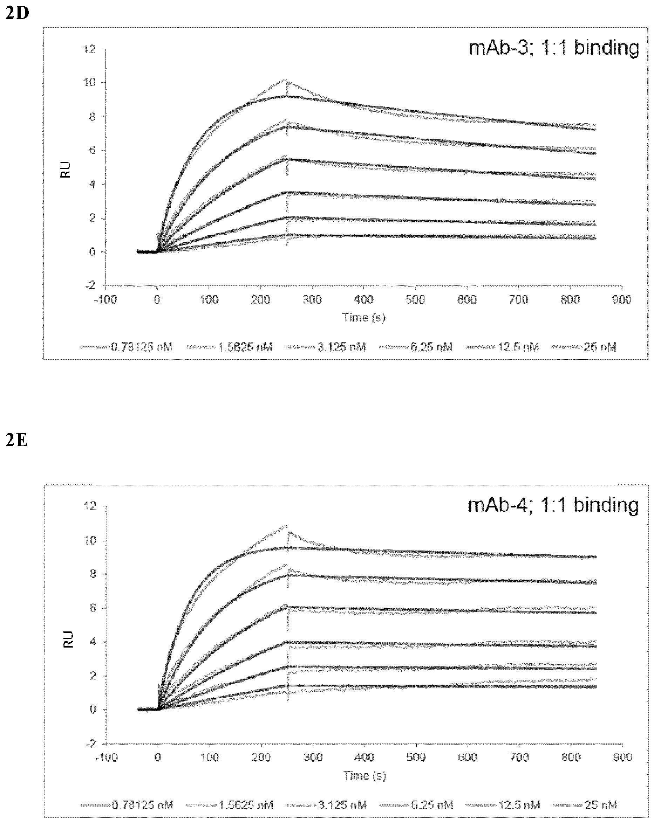

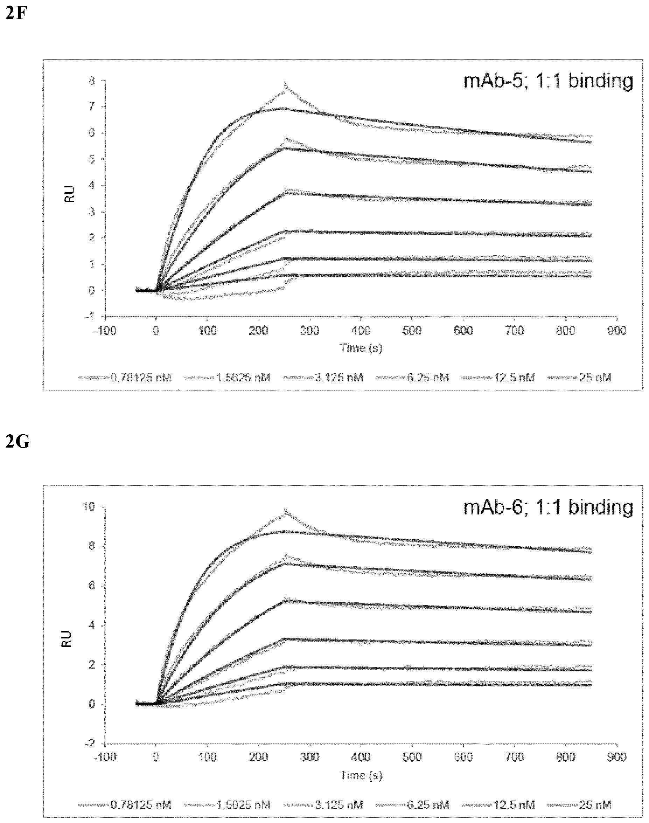

The results are shown in Figure 2 and Table 5.

**Table 5. Results of SPR analysis of antibody affinity**

| **Antibody** | **Quality Kinetics Chi² (RU²)** | **1:1 Binding Ka (1/Ms)** | **Kd (1/s)** | **KD (M)** |
|---|---|---|---|---|
| HA22 mAb | 1.91E-02 | 3.70E+05 | 8.93E-05 | 2.42E-10 |
| mAb-1 | 2.26E-02 | 4.34E+05 | 2.62E-04 | 6.04E-10 |
| mAb-2 | 2.30E-02 | 3.76E+05 | 1.77E-04 | 4.70E-10 |
| mAb-3 | 3.15E-02 | 5.67E+05 | 4.04E-04 | 7.13E-10 |
| mAb-4 | 5.75E-02 | 6. 85E+05 | 9.91E-05 | 1.45E-10 |
| mAb-5 | 3.76E-02 | 1.08E+06 | 4.11E-04 | 3.82E-10 |
| mAb-6 | 3.10E-02 | 6.99E+05 | 2.20E-04 | 3.15E-10 |

### Example 4 Study on binding of the antibodies to CD22-positive cell lines

Raji cells (having a relatively high expression of CD22), CA46 cells (having a relatively high expression of CD22), and REH cells (having a relatively medium expression of CD22) were selected, and the cell binding ability of the antibodies was evaluated by curves of mean fluorescence intensity versus concentration obtained through FACS.

### Reagents:

FACS Buffer: 2% FBS + PBS;
Goat anti-Human IgG (H+L) Secondary Antibody, FITC: Invitrogen, Catalogue number 31529; 4% PFA;
Allophycayanin (APC) AffiniPure F(ab')₂ Fragment Goat Anti-Human IgG, Fcy fragment specific, Jackson Immunoresearch, Catalogue number: 109-136-170.

Experimental steps were as follows:
1) Raji, CA46 and REH cells in logarithmic growth phase were harvested, and centrifuged at 1,000 rpm for 3 min.
2) The cells were washed twice with FACS Buffer (2% FBS + PBS), by centrifugation at 1,000 rpm for 3 min.
3) The cells were resuspended in FACS Buffer, and the cell density was adjusted to 2E6/mL.
4) The cell suspensions were added into 96 well round-bottom plates, at 50 µL per well, which were then placed on ice.
5) Each of the antibodies to be detected were 5-fold diluted serially to 8 concentrations: 100 µg/mL, 20 µg/mL, 4 µg/mL, 0.8 µg/mL, 0.16 µg/mL, 0.032 µg/mL, 0.0064 µg/mL and 0.00128 µg/mL.
6) The diluted antibodies were added into the wells containing cells at a 1:1 ratio by volume, to obtain final concentrations of 50 µg/mL, 10 µg/mL, 2 µg/mL, 0.4 µg/mL, 0.08 µg/mL, 0.016 µg/mL, 0.0032 µg/mL, 0.00064 µg/mL, and mixed with the cell suspensions, and the plates were then placed on ice in a 37 °C refrigerator and incubated for 30 min.
7) After the incubation, the cells were taken out and centrifuged at 2,000 rpm for 3 min.
8) The cells were washed twice with FACS Buffer, by centrifugation at 2,000 rpm for 3 min.
9) The cells were fixed with 2% PFA for 20 min at room temperature.
10) The cells were washed twice with FACS Buffer, by centrifugation at 2,000 rpm for 3 min.
11) The secondary antibodies (Goat anti-Human IgG (H+L) Secondary Antibody, FITC was used for Raji cells and REH cells; and Allophycayanin (APC) AffiniPure F(ab')₂ Fragment Goat Anti-Human IgG, Fcy fragment specific was used for CA46 cells) were formulated at 1:800 in buffers, and 100 µL of each were added into the cells, and the plates were then placed on ice in a 4 °C refrigerator and incubated for 30 min.
12) After the incubation, the cells were centrifuged at 2,000 rpm for 3 min.
13) Supernatants were discarded, and the cells were washed twice with FACS Buffer, by centrifugation at 2,000 rpm for 3 min.
14) The cells were suspended in 200 µL of FACS Buffer, and fluorescence intensity of FITC and fluorescence intensity of APC were detected.

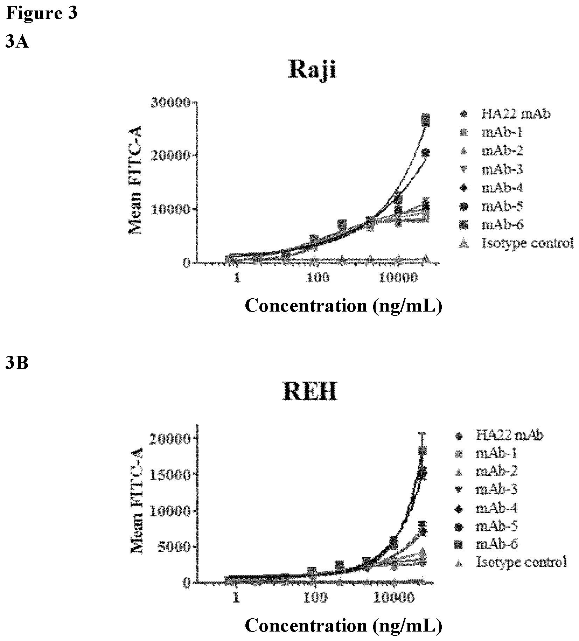

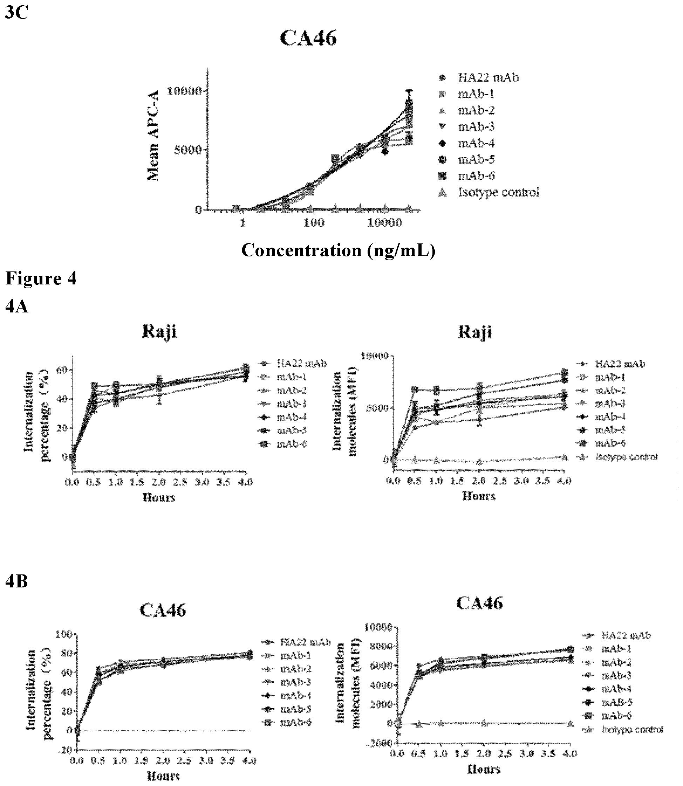

Results are shown in Figure 3 and Tables 6-1 to 6-3.

**Table 6-1. Detection results of binding of the antibodies to CD22 on the cell membrane of Raji cells by FACS**

| | **R2** | | **Top** | | **EC₅₀ (ng/mL)** |
|---|---|---|---|---|---|
| HA22 mAb | 0.9888 | HA22 mAb | 7506-8131 | HA22 mAb | 152.0 |
| mAb-1 | 0.9777 | mAb-1 | 7594-8558 | mAb-1 | 151.6 |
| mAb-2 | 0.9546 | mAb-2 | 5072-19663 | mAb-2 | 276.2 |
| mAb-3 | 0.9659 | mAb-3 | (very wide) | mAb-3 | ∼1.355e+022 |
| mAb-4 | 0.9572 | mAb-4 | 8407-13897 | mAb-4 | 206.3 |
| mAb-5 | 0.9467 | mAb-5 | (very wide) | mAb-5 | ∼1.362e+017 |
| mAb-6 | 0.9646 | mAb-6 | (very wide) | mAb-6 | ∼5.621e+013 |
| isotype control | 0.9878 | isotype control | 458.1-1079 | isotype control | 32619 |

As shown in panel 3A and Table 4, all the fitting parameters R2 reached above 0.95; the EC₅₀ value of the reference antibody HA22 mAb was 152 ng/mL, and the maximum fluorescence signal of the antibody was between 7506 and 8131 and had entered a plateau phase. The maximum fluorescence signal of mAb-1 was comparable to that of the reference antibody HA22 mAb, while the maximum fluorescence signals of the remaining mAbs were all higher than that of the reference antibody HA22 mAb, and the maximum fluorescence signals can be ranked as: mAb-6 > mAb-5 > mAb-3 > mAb-4 > mAb-2 > mAb-1 > HA22 mAb. Higher fluorescence intensity indicates that the humanized mAbs have higher binding potential on Raji cells than HA22 mAb. At a concentration of 10 ng/mL, a fluorescence signal already could be detected, indicating that binding of each antibody to Raji cells was achieved at 10 ng/mL. Antibodies mAb-3, mAb-5, and mAb-6 still did not reach a fluorescence signal plateau at 50 µg/mL, thereby resulting in inaccurate EC₅₀ values, but from the overall linearity evaluation, it can be determined that mAb-1 through mAb-6 had comparable or even stronger binding capacity to Raji cells.

**Table 6-2. Detection results of binding of the antibodies to CD22 on the cell membrane of REH cells by FACS**

| | **R2** | | **Top** | | EC₅₀ **(ng/mL)** |
|---|---|---|---|---|---|
| HA22 mAb | 0.9707 | HA22 mAb | 2364-2868 | HA22 mAb | 172.3 |
| mAb-1 | 0.9520 | mAb-1 | 1847-6579 | mAb-1 | 811.9 |
| mAb-2 | 0.9471 | mAb-2 | (very wide) | mAb-2 | ∼3.944e+025 |
| mAb-3 | 0.9494 | mAb-3 | (very wide) | mAb-3 | ∼2.2033+012 |
| mAb-4 | 0.9472 | mAb-4 | (very wide) | mAb-4 | ∼5.759e+013 |
| mAb-5 | 0.9855 | mAb-5 | (very wide) | mAb-5 | ∼4.166e+010 |
| mAb-6 | 0.9709 | mAb-6 | (very wide) | mAb-6 | ∼1.114e+099 |
| isotype control | 0.9250 | isotype control | (very wide) | isotype control | ∼8.250e+006 |

As shown in panel 3B and Table 6-2, on REH cells with moderate expression of CD22, mAb-1 through mAb-6 had comparable or even stronger binding capacity to REH cells compared to HA22 mAb.

**Table 6-3. Detection results of binding of the antibodies to CD22 on the cell membrane of CA46 cells by FACS**

| | **R2** | | **Top** | | EC₅₀ **(ng/mL)** |
|---|---|---|---|---|---|
| HA22 mAb | 0.9935 | HA22 mAb | 5724-6118 | HA22 mAb | 221.4 |
| mAb-1 | 0.9866 | mAb-1 | 6789-8271 | mAb-1 | 520.9 |
| mAb-2 | 0.9872 | mAb-2 | 5247-5846 | mAb-2 | 229.7 |
| mAb-3 | 0.9551 | mAb-3 | 2309-17864 | mAb-3 | 1611 |
| mAb-4 | 0.9804 | mAb-4 | 5170-5899 | mAb-4 | 152.5 |
| mAb-5 | 0.9662 | mAb-5 | (very wide) | mAb-5 | ∼2.015e+007 |
| mAb-6 | 0.9622 | mAb-6 | 3087-20780 | mAb-6 | 2752 |
| isotype control | 0.8198 | isotype control | (very wide) | isotype control | ∼4.590e+006 |

As shown in panel 3C and Table 6-3, on CA46 cells with high expression of CD22, mAb-1 through mAb-6 retained similar binding properties to that of HA22 mAb, all of which achieved high CA46 binding. According to the results of detection on all the three CD22 positive cell lines, humanized mAb-1 to mAb-6 can retain cell binding capacity comparable to or even superior to that of HA22 mAb.

### Example 5 Study on internalization efficiency of the antibodies

CD22 is an efficient target for internalization, so the internalization efficiency of an antibody against CD22 is often the most important index of evaluation for the development of immunotoxins and ADCs. To evaluate the internalization efficiency of the humanized antibodies i.e. mAb-1 to mAb-6, three CD22 positive cell lines (Raji, CA46 and REH cells) were selected for the evaluation and the internalization efficiency of the antibodies was compared with that of the reference antibody HA22 mAb.

### Reagents:

FACS Buffer: 2% FBS + PBS;
Goat anti-Human IgG (H+L) Secondary Antibody, FITC: Invitrogen, Catalogue number 31529; 4% PFA;
Allophycayanin (APC) AffiniPure F(ab')₂ Fragment Goat Anti-Human IgG, Fcy fragment specific, Jackson Immunoresearch, Catalogue number: 109-136-170.

Experimental steps were as follows:
1) Raji, CA46 and REH cells in logarithmic growth phase were harvested, and centrifuged at 1,000 rpm for 3 min.
2) The cells were washed twice with FACS Buffer, by centrifugation at 1,000 rpm for 3 min.
3) The cells were resuspended in FACS Buffer, and the cell density was adjusted to 5E6/mL.
4) The cell suspensions were added into 96 well round-bottom plates, at 100 µL per well, which were then placed on ice.
5) 100 µg/mL stock solution of an antibody to be tested was prepared, and the solution was added to the wells of the plates, at 100 µL per well, to obtain an antibody concentration of 50 µg/mL; the cells in the plates and the solution were mixed, and then the plates were placed on ice in a 4 °C refrigerator and incubated for 30 min.
6) After the incubation, the plates were taken out and the cells were centrifuged at 2,000 rpm at 4 °C for 3 min.
7) The cells were washed twice with FACS Buffer, by centrifugation at 2,000 rpm at 4 °C for 3 min.
8) One plate was used as the "0 h plate": the cells therein were fixed with 2% PFA for 20 min at room temperature, and then were washed twice with FACS Buffer (2,000 rpm, 3 min), suspended in FACS Buffer, and placed at 4 °C for subsequent secondary antibody labeling.
9) Other plates were used as the "0.5 h plate", the "1 h plate", the "2 h plate", and the "4 h plate": the cells in each well of the plates were suspended in 100 µL of FACS Buffer, and the plates were placed at a 37 °C constant temperature incubator for stationary culture and the culture time was recorded.
10) The plates were taken out at 0.5 h, 1 h, 2 h and 4 h respectively.
11) All the plates were centrifuged at 2,000 rpm at 4 °C for 3 min.
12) The secondary antibodies were formulated in buffers; and 100 µL of each were added into the cells and the plates were then placed on ice in a 4 °C refrigerator and incubated for 30 min.
13) After the incubation, the cells were centrifuged at 2,000 rpm 4 °C for 3 min.
14) Supernatants were discarded, and the cells were washed twice with FACS Buffer by centrifugation at 2,000 rpm 4 °C for 3 min.
15) The cells were suspended in 200 µL of FACS Buffer, and were detected.

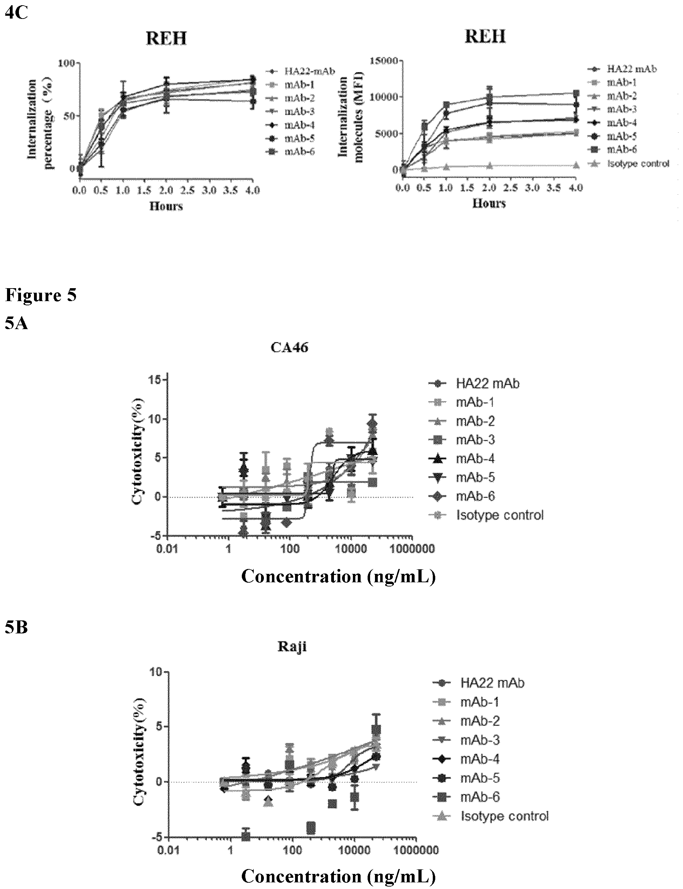

Results are shown in Figure 4.

At 0 h, no endocytosis occured, the antibodies were 100% concentrated on the cell surface; and the antibodies were endocytosed into the cells over time and the amounts of the antibodies on the membrane surface which could be detected decreased. The lower antibody signal is detected, the more endocytosis occurs. As shown in Figure 4, no significant difference in the endocytosis rates was found between mAb-1 to mAb-6 and HA22 mAb when endocytosis percentages of the antibodies into the Raji, CA46 and REH cells were evaluated, with maximal endocytosis efficiency achieved at 0.5 h on Raji and CA46 cells and at 1 h on REH cells. Although the 7 antibodies showed comparable endocytosis percentages, a large difference in the number of endocytosed molecules could be found by analysis of the mean fluorescence intensity values. On the CA46 cells, the antibodies achieved essentially identical number of endocytosed molecules, while on the Raji and REH cells, the numbers of endocytosed molecules of the humanized antibodies mAb-1 to mAb-6 were significantly higher than that of the reference antibody HA22 mAb, and could be ranked from high to low as follows: mAb-6 > mAb-5 > mAb-4 > mAb-3 > mAb-2 = mAb-1 = HA22 mAb.

### Example 6 Detection of antibody-dependent cell-mediated cytotoxicity (ADCC) of the antibodies

To evaluate whether the candidate antibodies have antibody-dependent cell-mediated cytotoxicity, three tumor cell lines, i.e., Raji, CA46 and REH cell lines having different CD22 expression levels were selected as target cells and PBMC as effector cells for ADCC assay.

### Reagent:

R-10 medium: phenol red-free RPMI 1640 + 10% inactivated FBS + 2mM Glutamax;
R-2 medium: phenol red-free RPMI 1640 + 2% inactivated FBS + 2mM Glutamax;
Cytotox Glo^{™} Cytotoxity Assay kit (Promega, Catalog number: G9291).

Experimental steps were as follows:
1) PBMCs were thawed one day in advance.
2) On the day of the experiment, the thawed PBMCs were harvested, centrifuged at 200 g for 15 min, and the supernatant was discarded. The cells were resuspended in 2 mL of fresh R-10 medium, counted, and the cell density was adjusted to 5E6 cells/mL. The cell suspension was added into plates, at 50 µL per well to obtain 250000 cells per well, or R-10 medium was added.
3) An antibody to be detected was diluted to 200 µg/mL, 40 µg/mL, 8 µg/mL, 1.6 µg/mL, 0.32 µg/mL, 0.064 µg/mL, 0.0128 µg/mL, and 0.00256 µg/mL with R-2 medium, and added into the plates, at 25 µL per well to obtain final concentrations of 50 µg/mL, 10 µg/mL, 2 µg/mL, 0.4 µg/mL, 0.08 µg/mL, 0.016 µg/mL, 0.0032 µg/mL, and 0.00064 µg/mL.
4) The Raji, CA46 and REH cells were diluted to 2E5 cells/mL with R-10 medium, and added into the plates, at 25 µL per well, to achieve an effector-to-target ratio of 50: 1.
5) The plates were incubated at 37 °C, 5% CO₂ for 4 h, and the color developed and was detected according to the instructions in the Cytotox Glo^{™} Cytotoxity Assay kit.

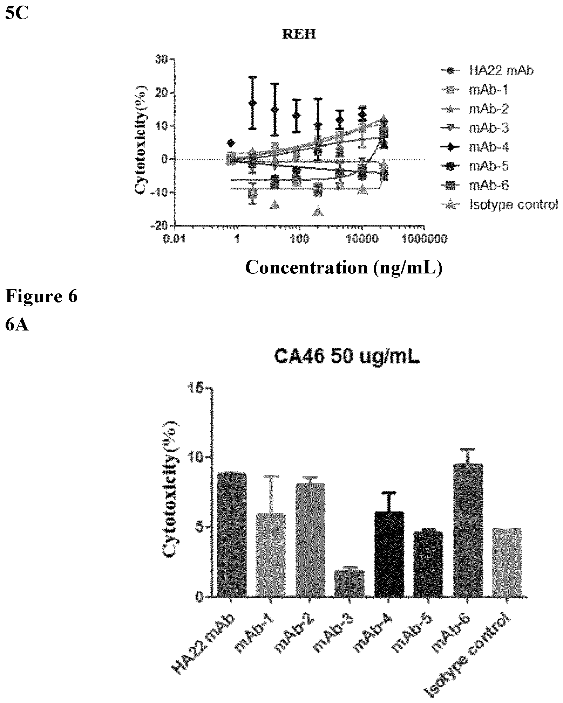

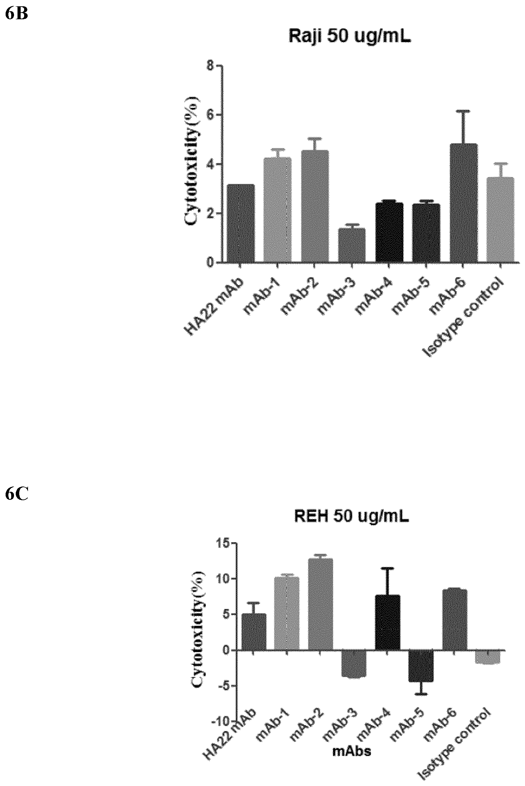

Results are shown in Figures 5 and 6.

As shown in Figure 5, all the 7 mabs had no significant ADCC effects on the tumor cells with different CD22 expression levels, and a dose-response dependence curve could not be fitted; while they exhibited slight ADCC effects at the high dose of 50 µg/mL.

The cytotoxicities observed at the dose of 50 µg/mL were analyzed, and as shown in Figure 6, mAb-3 showed a relatively low cytotoxicity, having no difference from an isotype control. Antibodies mAb-1, mAb-2, mAb-4, mAb-5, and mAb-6 showed no different cytotoxicities from HA22 on the Raji and CA46 cells having high CD22 expression levels. However, mAb-2 showed a slightly more potent cytotoxicity than HA22 mAb on REH cells having a lower CD22 expression level. In conclusion, no significant ADCC effect was shown by the 7 IgG1 antibodies.

The above description of the embodiments of the present invention is not intended to limit the present invention, and those skilled in the art may make various changes and modifications to the present invention without departing from the spirit of the present invention, which should fall within the scope of the appended claims.

## Claims

1. An isolated anti-CD22 antibody or antigen-binding fragment thereof, comprising a heavy chain variable region (VH) and a light chain variable region (VL), wherein the heavy chain variable region (VH) and the light chain variable region (VL) comprise respectively:
(1) H-CDR1 having an amino acid sequence as shown in SEQ ID NO: 19, H-CDR2 having an amino acid sequence as shown in SEQ ID NO: 25, and H-CDR3 having an amino acid sequence as shown in SEQ ID NO: 21; and, L-CDR1 having an amino acid sequence as shown in SEQ ID NO: 22, L-CDR2 having an amino acid sequence as shown in SEQ ID NO: 23, and L-CDR3 having an amino acid sequence as shown in SEQ ID NO: 24; or
(2) H-CDR1 having an amino acid sequence as shown in SEQ ID NO: 19, H-CDR2 having an amino acid sequence as shown in SEQ ID NO: 26, and H-CDR3 having an amino acid sequence as shown in SEQ ID NO: 21; and, L-CDR1 having an amino acid sequence as shown in SEQ ID NO: 22, L-CDR2 having an amino acid sequence as shown in SEQ ID NO: 23, and L-CDR3 having an amino acid sequence as shown in SEQ ID NO: 24.

2. The antibody or antigen-binding fragment thereof according to claim 1, wherein the heavy chain variable region comprises an amino acid sequence as shown in SEQ ID NO: 4, SEQ ID NO: 3, SEQ ID NO: 6, or SEQ ID NO: 5, or an amino acid sequence having at least 75% identity to the amino acid sequence as shown in SEQ ID NO: 4, SEQ ID NO: 3, SEQ ID NO: 6, or SEQ ID NO: 5; and/or,
the light chain variable region comprises an amino acid sequence as shown in SEQ ID NO: 2, SEQ ID NO: 7, or SEQ ID NO: 8, or an amino acid sequence having at least 75% identity to the amino acid sequence as shown in SEQ ID NO: 2, SEQ ID NO: 7, or SEQ ID NO: 8.

3. The antibody or antigen-binding fragment thereof according to claim 1 or 2, wherein the heavy chain variable region and the light chain variable region comprise respectively:
(1) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 2 or an amino acid sequence having at least 75% identity to the amino acid sequence as shown in SEQ ID NO: 2;
(2) an amino acid sequence as shown in SEQ ID NO: 4 or an amino acid sequence having at least 75% identity to the amino acid sequence as shown in SEQ ID NO: 4; and, an amino acid sequence as shown in SEQ ID NO: 7 or an amino acid sequence having at least 75% identity to the amino acid sequence as shown in SEQ ID NO: 7;
(3) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 7 or an amino acid sequence having at least 75% identity to the amino acid sequence as shown in SEQ ID NO: 7;
(4) an amino acid sequence as shown in SEQ ID NO: 3 or an amino acid sequence having at least 75% identity to the amino acid sequence as shown in SEQ ID NO: 3; and, an amino acid sequence as shown in SEQ ID NO: 8 or an amino acid sequence having at least 75% identity to the amino acid sequence as shown in SEQ ID NO: 8;
(5) an amino acid sequence as shown in SEQ ID NO: 6 or an amino acid sequence having at least 75% identity to the amino acid sequence as shown in SEQ ID NO: 6; and, an amino acid sequence as shown in SEQ ID NO: 8 or an amino acid sequence having at least 75% identity to the amino acid sequence as shown in SEQ ID NO: 8; or
(6) an amino acid sequence as shown in SEQ ID NO: 5 or an amino acid sequence having at least 75% identity to the amino acid sequence as shown in SEQ ID NO: 5; and, an amino acid sequence as shown in SEQ ID NO: 8 or an amino acid sequence having at least 75% identity to the amino acid sequence as shown in SEQ ID NO: 8.

4. The antibody or antigen-binding fragment thereof according to any one of claims 1 to 3, wherein the antibody is a murine antibody, a chimeric antibody or a fully or partially humanized antibody;
alternatively, the antibody is in the form of a scFv, dsFv, (dsFv)₂, Fab, Fab', F(ab')₂ or Fv antibody; preferably, the antibody is a monoclonal antibody or a single chain antibody;
preferably, the antibody comprises a human or murine constant region, preferably a human or murine heavy chain constant region and/or light chain constant region;
preferably, the antibody comprises a heavy chain and a light chain; more preferably, the antibody comprises a heavy chain constant region selected from the group consisting of IgG, IgA, IgM, IgD and IgE and/or a kappa or lambda type light chain constant region.

5. The antibody or antigen-binding fragment thereof according to any one of claims 1 to 4, wherein the antibody is a monoclonal antibody, preferably a murine, chimeric or humanized monoclonal antibody;
preferably, the light chain of the monoclonal antibody is of a kappa type;
more preferably, the monoclonal antibody is an IgG1 antibody.

6. A nucleic acid molecule comprising a nucleotide sequence encoding the anti-CD22 antibody or antigen-binding fragment thereof according to any one of claims 1 to 5, or encoding a heavy chain CDR, a light chain CDR, a heavy chain variable region, a light chain variable region, a heavy chain or a light chain comprised in the anti-CD22 antibody or antigen-binding fragment thereof according to any one of claims 1 to 5.

7. A vector comprising the nucleic acid molecule according to claim 6.

8. A host cell comprising the nucleic acid molecule according to claim 6 and/or the vector according to claim 7, or transformed or transfected with the nucleic acid molecule according to claim 6 and/or the vector according to claim 7.

9. A composition comprising the antibody or antigen-binding fragment thereof according to any one of claims 1 to 5, the nucleic acid molecule according to claim 6, the vector according to claim 7, and/or the host cell according to claim 8.

10. An immunoconjugate comprising the antibody or antigen-binding fragment thereof according to any one of claims 1 to 5 and a partner molecule which is a therapeutic agent.

11. Use of the antibody or antigen-binding fragment thereof according to any one of claims 1 to 5, the nucleic acid molecule according to claim 6, the vector according to claim 7, the host cell according to claim 8, the composition according to claim 9, and/or the immunoconjugate according to claim 10 in the manufacture of a medicament for treating a disease.

12. A kit comprising the antibody or antigen-binding fragment thereof according to any one of claims 1 to 5, the nucleic acid molecule according to claim 6, the vector according to claim 7, the host cell according to claim 8, the composition according to claim 9, and/or the immunoconjugate according to claim 10.

13. A method for treating a disease, the method comprising administering to a subject in need thereof the antibody or antigen-binding fragment thereof according to any one of claims 1 to 5, the nucleic acid molecule according to claim 6, the vector according to claim 7, the host cell according to claim 8, the composition according to claim 9, and/or the immunoconjugate according to claim 10.
